(19) **Europäisches Patentamt** / **European Patent Office** / **Office européen des brevets**

(11) **EP 2 599 748 B1**

(12) **EUROPEAN PATENT SPECIFICATION**

(45) Date of publication and mention
of the grant of the patent:
**20.05.2020 Bulletin 2020/21**

(51) Int Cl.:
*C01B 37/02* (2006.01)    *B01J 29/40* (2006.01)
*B01J 37/06* (2006.01)    *C07D 201/04* (2006.01)

(21) Application number: **12194714.7**

(22) Date of filing: **29.11.2012**

(54) **Method for producing zeolite molded article and method for producing epsilon-caprolactam**

Verfahren zur Herstellung eines Zeolithformteils und Verfahren zur Herstellung von Epsilon-Caprolactam

Procédé de production d'article moulé à base de zéolite et procédé de production d'epsilon-caprolactame

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.11.2011 JP 2011261481**

(43) Date of publication of application:
**05.06.2013 Bulletin 2013/23**

(73) Proprietor: **Sumitomo Chemical Company, Limited**
**Tokyo 104-8260 (JP)**

(72) Inventors:
• **Sugita, Keisuke**
**Niihama-shi, Ehime 792-8521 (JP)**
• **Hoshino, Masahiro**
**Niihama-shi, Ehime 792-8521 (JP)**
• **Kasai, Koji**
**Niihama-shi, Ehime 792-8521 (JP)**

(74) Representative: **Vossius & Partner Patentanwälte Rechtsanwälte mbB**
**Siebertstrasse 3**
**81675 München (DE)**

(56) References cited:
**EP-A1- 0 576 295    EP-A2- 1 614 657**
**EP-A2- 2 157 080    US-A- 4 222 995**

**Description**

BACKGROUND OF THE INVENTION

The present invention relates to a method for producing a zeolite molded article. The present invention also relates to a method for producing $\varepsilon$-caprolactam from cyclohexanone oxime using the zeolite molded article as a catalyst.

Conventionally, a method of Beckmann rearrangement reaction of cyclohexanone oxime in a gaseous phase in the presence of zeolites using the zeolites as a solid catalyst, has been proposed as one of methods for producing $\varepsilon$-caprolactam (see, e.g., JP 2-250866 A and JP 2-275850 A). Such zeolite is known to be used after molding, and as a method for producing such a zeolite, there has been proposed a method comprising subjecting, for example, a mixture of a silicon compound, water, and a quaternary ammonium hydroxide to a hydrothermal synthesis reaction, filtering off the solid from the reaction mixture obtained, washing the solid with water until the pH of the filtrate becomes around pH 7, and then molding the washed solid, followed by calcination (see JP 6-72706 A).

SUMMARY OF THE INVENTION

However, in the conventional method, mechanical strength of the zeolite molded article was not necessarily satisfactory. Accordingly, an object of the present invention is to provide a method for producing a zeolite molded article excellent in mechanical strength. In addition, another object of the present invention is to provide a method for producing $\varepsilon$-caprolactam in a good selectivity comprising reacting cyclohexanone oxime in a good conversion rate using, as a catalyst, a zeolite molded article obtained by such a producing method.

As a result of intensive studies for the purpose of achieving the above objects, the present inventors have accomplished the present invention, which is as set out in the claims.

Effect of the Invention

According to the present invention, a zeolite molded article having an excellent mechanical strength can be produced, and using as a catalyst the zeolite molded article obtained by such a producing method, $\varepsilon$-caprolactam can be produced in a good selectivity by subjecting cyclohexanone oxime to Beckmann rearrangement reaction in a gaseous phase so that cyclohexanone oxime can be reacted in a good conversion rate.

DETAILED DESCRIPTION OF THE PREFERRED EMBODIMENTS

Hereinafter, the present invention will be described in detail. A zeolite, as a target of the present invention, in the zeolite molded article includes silicon and oxygen as elements constituting a skeleton thereof and may be a crystalline silica having a skeleton substantially constituted with silicon and oxygen, or may be a crystalline metallosilicate or the like further including other elements as elements constituting a skeleton thereof. In the case of the crystalline metallosilicate or the like, other elements that may exist in addition to silicon and oxygen include, for example, Be, B, Al, Ti, V, Cr, Fe, Co, Ni, Cu, Zn, Ga, Ge, Zr, Nb, Sb, La, Hf, Bi, etc., and two or more of them may be included if necessary. Further, the atomic ratio of silicon to these elements is preferably 5 or more, and more preferably 500 or more.

Although various structures are known for the above-mentioned zeolite, a zeolite having a pentasil type structure is preferable among them, and a zeolite having an MFI structure is especially preferable. Among the zeolites having an MFI structure, silicalite-1 is preferred. The silicalite-1 that is an MFI-zeolite having high silicate properties can be synthesized by hydrothermal synthesis of a mixture of a tetraalkyl orthosilicate, water, and tetra-n-propylammonium hydroxide under an autogenous pressure.

In the present invention, the step (1) comprises subjecting a mixture including a silicon compound, water, and a quaternary ammonium hydroxide to a hydrothermal synthesis reaction.

Examples of the silicon compound used in the step (1) include amorphous silica such as colloidal silica, silica gel, fumed silica, etc.; alkali silicate such as sodium silicate, potassium silicate, etc.; and tetraalkyl orthosilicate such as tetramethyl orthosilicate, tetraethyl orthosilicate, tetrapropyl orthosilicate, tetrabutyl orthosilicate, etc., and two or more of them can also be used if necessary. In tetraalkyl orthosilicate the alkyl group can preferably have 1 to 6 carbon atoms. Among them, tetraalkyl orthosilicate is preferred, and tetraethyl orthosilicate is more preferred.

In the present invention, the quaternary ammonium hydroxide is used as a structure-directing agent. The structure-directing agent (template) refers to an organic compound that may be utilized to form a zeolite structure. The structure-directing agent may form a precursor of the zeolite structure by organizing polysilicic acid ions and polymetallosilicic acid ions around it (see Science and Engineering of Zeolite (in Japanese), published by Kodansha Scientific Ltd., (2000), p.33-34). The quaternary ammonium hydroxide used in the step (1) can include, for example, a compound represented by the following formula (I):

$$R^1R^2R^3R^4N^+OH^-  \qquad \text{(I)}$$

wherein $R^1$, $R^2$, $R^3$, and $R^4$ each independently represents an alkyl group, an alkenyl group, an aralkyl group, or an aryl group. The alkyl group can, e.g., have 1 to 6 carbon atoms. The alkenyl group can, e.g., have 1 to 6 carbon atoms. The aralkyl group can, e.g., have 6 to 16 carbon atoms. The aryl group can, e.g., have 5 to 10 carbon atoms.

The quaternary ammonium hydroxide represented by the formula (I) is preferably a tetraalkylammonium hydroxide, and examples of the tetraalkylammonium hydroxide include tetramethylammonium hydroxide, tetraethylammonium hydroxide, n-propyltrimethylammonium hydroxide, tetra-n-propylammonium hydroxide, tetra-n-butylammonium hydroxide, triethylmethylammonium hydroxide, tri-n-propylmethylammonium hydroxide, tri-n-butylmethylammonium hydroxide, and the like.

The molar ratio of water to silicon in the mixture of step (1) is preferably 5 to 100, more preferably 10 to 60.

The molar ratio of quaternary ammonium ions to silicon in the mixture of step (1) is preferably 0.1 to 0.6, more preferably 0.2 to 0.5.

The molar ratio of hydroxide ions to silicon in the mixture of step (1) is usually adjusted to 0.1 to 0.6, preferably to 0.2 to 0.5. The primary particle diameter of zeolite tends to become larger as the molar ratio of hydroxide ions to silicon in the mixture becomes smaller. Therefore, if such molar ratio is less than 0.1, the primary particle diameter of the obtained zeolite may become too large, leading to reduction in its outer surface area, because of which the catalytic activity may not be sufficient. In addition, if the molar ratio is more than 0.6, the particle diameter of the obtained zeolite may become too small and thus its handling characteristics such as filterability, etc. may not be good. The molar ratio of hydroxide ions to silicon in the mixture can be appropriately adjusted by controlling the amounts used of the silicon compound and the quaternary ammonium hydroxide.

The molar ratio of potassium to silicon in the mixture of step (1) is preferably adjusted to 0 to 0.1, more preferably to 0.04 to 0.1. The molar ratio of potassium to silicon in the mixture can be appropriately adjusted by, for example, controlling the amount used of the silicon compound, or managing the content of the potassium compound that may be included as an impurity in each raw material, particularly in the quaternary ammonium hydroxide.

When the mixture is prepared, other components in addition to the silicon compound, water and quaternary ammonium hydroxide may be used as raw materials if necessary. For example, a basic compound such as sodium hydroxide or potassium hydroxide may be mixed in order to adjust the concentration of hydroxide ions in the mixture. Also, for example, when a tetraalkylammonium hydroxide is used as the quaternary ammonium hydroxide, a tetraalkylammonium salt such as tetraalkylammonium bromide may be mixed in order to adjust the concentration of tetraalkylammonium ions in the mixture. Further, for example, in order to adjust the concentration of potassium ions in the mixture, a potassium compound such as potassium hydroxide may be mixed. When a zeolite containing elements other than silicon and oxygen described above is prepared, a compound containing an element other than silicon and oxygen may be mixed. With respect to elements other than silicon and oxygen that were previously exemplified, when a compound including these elements is mixed, the molar ratio of silicon to such an element in the mixture is preferably adjusted to 5 or more and more preferably to 500 or more.

The content of silicon contained in the mixture of step (1) can be determined, for example, by inductively coupled plasma optical emission spectrometry (ICP). The content of the tetraalkylammonium ions in the mixture can be determined, for example, by ion chromatography in the case where a tetraalkylammonium hydroxide is used as the quaternary ammonium hydroxide. In addition, the content of hydroxide ions in the mixture can be determined by subtracting the content of basic ions other than hydroxide ions that may be included as impurities in the quaternary ammonium hydroxide from the total content of basic ions determined by neutralization titration with an acid (e.g., 0.2 N hydrochloric acid).

In the step (1), the mixture is subjected to a hydrothermal synthesis reaction. The temperature in the hydrothermal synthesis is preferably 80 to 160°C, more preferably 100 to 140°C. Further, the reaction time for the hydrothermal synthesis is preferably 1 to 200 hours, more preferably 12 to 72 hours. The pressure in the hydrothermal synthesis is preferably in the range of 0.10 to 1.0 MPa, more preferably in the range of 0.11 to 0.50 MPa, as absolute pressure. The hydrothermal synthesis method includes, but is not limited to, for example, a method which is carried out by filling the mixture in a reaction vessel such as an autoclave and stirring it at the temperature conditions described above in a sealed state.

In the present invention, the step (2) comprises filtering the reaction mixture containing crystals obtained in the step (1), thereby to separate it into a concentrate containing crystals and a filtrate. As a filtration method, preferred is a membrane separation method using a microfiltration membrane (MF membrane) or an ultrafiltration membrane (UF membrane). The material and pore size of such MF membrane or UF membrane are appropriately set. The filtration manner may include a cross-flow filtration manner and a dead-end filtration manner, but the cross-flow filtration manner is preferable from the viewpoint that washing can be easily and efficiently performed. In addition, the filtration manner may be an external pressure filtration manner and an inner pressure filtration manner. The filtration may be performed by any of a pressurized filtration or a suction filtration, but a pressurized filtration is preferable. The pressure in the filtration is appropriately set, and the filtration may be carried out by any of a constant flow filtration to perform the filtration while keeping the amount of filtrate constant or a constant pressure filtration to perform the filtration while keeping the transmembrane pressure difference constant. The temperature in the filtration is preferably in the range of from room temperature to temperatures at the time of the hydrothermal synthesis reaction. In addition, the filtration time is preferably 0.1 to 30 hours. The pressure during filtration is preferably in the range of 0.01 to 1 MPa as an absolute pressure.

The content rate of the crystals contained in the reaction mixture obtained in the step (1) is preferably 1 to 50% by mass as a mass of crystals excluding a portion of a structure-directing agent that has been incorporated into the crystals. The content rate of the crystals contained in the concentrate obtained in the step (2) is preferably 5 to 80% by mass as a mass of crystals excluding a portion of a structure-directing agent that has been incorporated into the crystals.

Since the filtrate obtained in the step (2) includes usually silicic acid and oligomer as the active components and the unreacted quaternary ammonium hydroxide, etc., an environmentally friendly producing process with less waste can be devised by mixing at least a portion of the filtrate as a raw material in the step (1) so that an effective use becomes possible by its recycling use. In the case where an alcohol such as ethanol, etc. is contained in the filtrate, it is desirable to remove a part or whole of the alcohol by a procedure such as distillation and the like before recycling the filtrate when an alcohol such as ethanol and the like is generated by hydrothermal synthesis after mixing with, for example, a tetraalkyl orthosilicate such as tetraethyl orthosilicate and the like as a silicon compound. In the recycling use of the filtrate, preferred is to manage it so that carbon dioxide does not dissolve into the solution during storage. Specifically, it is preferable to seal the filtrate with a nitrogen gas or with an air from which carbon dioxide has been removed and to store the filtrate.

In the present invention, the step (3) comprises washing the concentrate containing the crystals obtained in the step (2), with water until the pH at 25°C of the washed solution obtained by washing becomes 8.5 to 9.5. Preferably, such washing process is carried out until the pH at 25°C of the washed solution obtained by washing becomes 8.8 to 9.2. Usually, the concentrate containing the crystals obtained in the step (2) is in a high alkaline region due to the hydroxide ions contained in the concentrate, but by performing such washing process until the pH at 25°C of the washed solution obtained by washing becomes within the range described above, it is possible to effectively improve the mechanical strength of the zeolite molded article which is finally obtained. If the washing process is performed until the pH at 25°C of the washed solution obtained by washing becomes less than 8.5, such washing might be an excessive washing, and if the washing process is stopped at a pH of greater than 9.5, such washing might be lack of washing. In both cases, the mechanical strength of each of the finally obtained zeolite molded articles tends to reduce. In order to wash the zeolite molded article with water until the pH at 25°C of the washed solution obtained by washing becomes 8.5 to 9.5, the pH can be adjusted within the range described above by, for example, controlling the amount of water for washing.

Examples of the washing method include (A) a washing filtration method with water in a cross-flow manner by the addition of water to the concentrate; (B) a washing filtration method with water in a dead-end filtration manner by the addition of water to the concentrate; (C) a method comprising mixing the concentrate with water and separating the supernatant by decantation; and the like. In the method (A), the washing filtration is carried out until the pH at 25°C of the washing filtrate obtained by washing filtration becomes 8.5 to 9.5. The method (A) may be a washing method wherein the addition of water to the concentrate and the washing filtration in a cross-flow manner are continuously performed or may be a washing method wherein water supply and washing filtration are repeated such that water is added to the concentrate, washing filtration is performed in a cross-flow manner, water is newly added, and then washing filtration is performed. When the addition of water to the concentrate and the washing filtration in a cross-flow manner are continuously performed in the method (A), the pH of the washing filtrate may be detected continuously or the pH of a portion of the washing filtrate extracted during the washing filtration may be measured. The continuous detection of the pH of the washing filtrate may be preferably conducted using a pH sensor, etc. in a pipe where the washing filtrate after being withdrawn from a filtration device flows. In the case where the pH of a portion of the washing filtrate withdrawn during washing is measured, it is desirable to withdraw a portion of the washing filtrate which has been withdrawn from a filtration device and flows in the pipe. If water supply/washing filtration were repeated in the method (A), the pH of the washing filtrate may be continuously detected, or the pH of a portion withdrawn from the washing filtrate during washing filtration may be measured, or the pH of the washing filtrate collected in a reservoir and the like at each water supply/washing filtration may be measured. The continuous detection of the pH of the washing filtrate is preferably conducted using a pH sensor, etc. in a pipe in which the washing filtrate after being withdrawn from a filtration device flows. In the case where the pH of a portion of the washing filtrate withdrawn during washing filtration is measured, it is preferable to withdraw a portion of the washing filtrate which has been withdrawn from a filtration device and flows in the pipe.

In the method (B), washing filtration is carried out until the pH at 25°C of the washing filtrate obtained by washing filtration becomes 8.5 to 9.5. The method (B) may be a washing method wherein the addition of water to the concentrate and the washing filtration in a dead-end filtration manner are continuously performed or may be a washing method wherein water supply and washing filtration are repeated such that water is added to the concentrate, washing filtration is performed in a dead-end filtration manner, water is newly added, and then washing filtration is performed. When the addition of water to the concentrate and the washing filtration in a dead-end filtration manner are continuously performed in the method (B), the pH of the washing filtrate may be detected continuously or the pH of a portion of the washing filtrate withdrawn during the washing filtration may be measured. The continuous detection of the pH of the washing filtrate may be preferably conducted using a pH sensor, etc. in a pipe in which the washing filtrate after being withdrawn from a filtration device flows. In the case where the pH of a portion of the washing filtrate withdrawn during washing filtration is measured, it is desirable to withdraw a portion of the washing filtrate which has been withdrawn from a filtration device and flows in the pipe. If water supply/washing filtration were repeated in the method (B), the pH of the washing filtrate may be continuously detected, or the pH of a portion withdrawn from the washing filtrate during washing filtration may be measured, or the pH of the washing filtrate collected in a reservoir at each water supply/washing filtration may be measured. The continuous detection of the pH of the washing filtrate is preferably conducted using a pH sensor, etc. in a pipe in which the washing filtrate after being withdrawn from a filtration device flows. In the case where the pH of a portion of the washing filtrate withdrawn during washing filtration is measured, it is desirable to withdraw a portion of the washing filtrate which has been withdrawn from a filtration device and flows in the pipe.

In the method (C), washing filtration is carried out until the pH at 25°C of the supernatant becomes 8.5 to 9.5. In the method (C), there is employed a washing method of repeating water supply/decantation, the method comprising mixing usually the concentrate and water under stirring, separating the supernatant by decantation, mixing the precipitate with newly added water under stirring, and separating the supernatant by decantation. For the supernatant obtained by decantation in the method (C), washing is conducted until the pH at 25°C of the supernatant becomes 8.5 to 9.5.

Among the methods (A) to (C), the method (A) is preferable in that washing can be performed easily and efficiently. That is, step (3) is constituted by a step (3') comprising the washing filtration with water in a cross-flow manner of a concentrate containing crystals obtained in the step (2) until the pH at 25°C of the washing filtrate becomes 8.5 to 9.5, and it is preferable to subject the washed crystals obtained in the step (3') to the step (4) which is described later.

The temperature for washing in the step (3) is preferably within the range of from room temperature to hydrothermal synthesis reaction temperature. Further, the washing time is preferably 0.1 to 30 hours. The pressure in the washing step is preferably in the range of 0.01 to 1 MPa as an absolute pressure.

When washing filtration is carried out in the step (3), the washing filtration method includes preferably a membrane separation method using MF membrane or UF membrane. The material and pore size of such MF membrane or UF membrane are appropriately set. The washing filtration manner may include an external pressure filtration manner and an inner pressure filtration manner. The washing filtration may be performed by any of a pressurized filtration or a suction filtration, but a pressurized filtration is preferable. The pressure in the washing filtration is appropriately set, and may include any of a constant flow filtration to perform the filtration while keeping the amount of filtrate constant or a constant pressure filtration to perform the filtration while keeping the transmembrane pressure difference constant.

The washed crystals obtained in the step (3) may be collected as a slurry or as a dried product after drying if necessary, and it is preferable to collect such washed crystals as a slurry. If the washed crystals are collected as a slurry, the content ratio of the crystals contained in the slurry is preferably 10 to 70% by mass as a mass of crystals excluding a portion of the structure-directing agent incorporated into the crystals, more preferably 30 to 60% by mass. The content ratio of the crystals contained in the slurry is adjustable by adjusting, for example, the amount of water to be added at the time of washing and the amount of the washed solution to be withdrawn. The content ratio of the crystals contained in the slurry may be adjusted by applying a concentration process, such as distillation, centrifuge, etc., thereto or adding water thereto as needed so that it is within the range described above.

Since the washed solution obtained in the step (3), particularly the washed solution at the initial stage of washing, includes usually silicic acid and oligomers as active components, and unreacted quaternary ammonium hydroxide, an environmentally friendly producing process with less waste can be devised by mixing in the step (1) at least a portion of the washed solution obtained in the step (3) as a raw material so that an effective use becomes possible by its recycling use. In the case where an alcohol such as ethanol, etc. is contained in the washed solution, it is desirable to remove a part or whole of the alcohol by a procedure such as distillation, etc. before recycling the washed solution when an alcohol such as ethanol and the like is generated by hydrothermal synthesis after mixing with, for example, a tetraalkyl orthosilicate such as tetraethyl orthosilicate and the like as a silicon compound. In the recycling use of the washed solution, it is preferable to manage it so that carbon dioxide does not dissolve into the solution during storage. Specifically, preferred is to seal the washed solution with a nitrogen gas or an air from which carbon dioxide has been removed and to store the washed solution.

As the step (4) in the present invention, it comprises molding the washed crystals obtained in the step (3). In molding the crystals of a zeolite, for example, an extrusion, compression, tablet, flow, rolling, or spray molding can be used, and the crystals can be molded into a desired shape such as sphere, column, plate, ring, clover, etc. As for a zeolite molded article, a shape may be chosen depending on its use and a molding method may be chosen depending on its shape. For example, when the zeolite molded article is used as a catalyst for fixed bed type reaction, a pellet-like molding such as column and cylinder is produced by adopting a method such as extrusion or tablet molding or the like, and when the zeolite molded article is used as a catalyst for fluidized bed type reaction or moving bed type reaction, fine particle moldings such as spheres are produced by spray drying the slurry. In particular, the method for producing a zeolite molded article of the present invention is advantageously adopted when performing such a molding by spray drying the slurry containing the washed crystals obtained in the step (3). In addition, this zeolite molded article may be comprised of substantially only zeolite or may be comprised of zeolite and other components. For example, the zeolite molded article may be those obtained by molding substantially only zeolite, those obtained by mixing zeolite with binders and reinforcing materials or the like, followed by molding, or those obtained by supporting zeolite on a carrier, but the method for producing a zeolite molded article according to the present invention is advantageously adopted when substantially only zeolite is molded. In addition, the particle size of the zeolite molded article is usually 5 mm or less, preferably 3 mm or less. As the zeolite to be molded, it is preferable to use a zeolite having a primary particle diameter of 5 $\mu$m or less, more preferably 1 $\mu$m or less.

The step (5) of the present invention comprises calcining the molded article obtained in the step (4). This calcination is usually carried out preferably under an oxygen-containing gas atmosphere, for example, under an air atmosphere or under an atmosphere of a mixed gas of air and nitrogen, at a temperature of 400 to 600°C. In addition, calcination may be performed under an inert gas atmosphere such as nitrogen and the like before and after such calcination under an oxygen-containing gas atmosphere.

In the present invention, other processing as specified in claim 1 is applied to the calcined product obtained in the step (5) .

That is, the present invention includes as a step (6) a step of contacting the calcined product obtained in the step (5) with water vapor. Such contact of the calcined product with water vapor can more effectively improve the mechanical strength of the zeolite molded article that is finally obtained.

When the contact processing of the calcined product with water vapor is performed, the contact temperature is preferably 30 to 200°C, more preferably 50 to 150°C. The contact time is usually 5 minutes to 72 hours. Such contact processing may be repeated if necessary. Examples of the contact processing include a method in which water vapor flows through a column packed with the calcined product; a method to retain the calcined product under an atmosphere of water vapor; and the like. A basic gas such as ammonia, methylamine and the like, an oxygen-containing gas, or an inert gas may coexist together with the water vapor. If the basic gas is allowed to coexist, the basic gas is used usually in an amount of 0.01 to 1 mole, preferably 0.1 to 0.5 moles, based on 1 mole of water vapor. The calcined product after the processing may be washed with an acid such as hydrochloric acid and the like or water, and when the calcined product is washed with an acid, it may be further washed with water. The calcined product after contacting with water vapor can also be further calcined if necessary.

The thus obtained zeolite molded article has a high mechanical strength, and can be used in various applications including a catalyst for organic synthesis reactions. Among them, such a zeolite molded article can be used suitably as a catalyst for producing $\varepsilon$-caprolactam by Beckmann rearrangement reaction of cyclohexanone oxime in a gaseous phase.

In the present invention, from the viewpoint of improving a catalytic performance as a catalyst for organic synthesis reactions, the calcined product after contacting with water vapor obtained in the step (6) is subjected to a contact processing with an aqueous solution comprising at least one compound selected from the group consisting of ammonia and an ammonium salt, in particular in view of the fact that the mechanical strength of the zeolite molded article finally obtained becomes good,

The present invention,

further includes as a step (7) a step to apply the calcined product after contacting with water vapor obtained in the step (6) to a contact processing with an aqueous solution comprising at least one compound selected from the group consisting of ammonia and an ammonium salt.

The aqueous solution includes preferably at least ammonia, and the ammonia concentration in the aqueous solution is preferably 2 to 30% by mass, more preferably 5 to 25% by mass. Examples of the ammonium salt used in the contact processing includes ammonium chloride, ammonium sulfate, ammonium nitrate, ammonium acetate, ammonium phosphate, ammonium carbonate, ammonium fluoride, ammonium bromide and the like, among which ammonium nitrate and ammonium acetate are preferred. When an ammonium salt is allowed to be contained in the aqueous solution, its content is usually 0.001 to 1 moles, preferably 0.01 to 0.1 moles, based on 1 mole of ammonia.

The aqueous solution may contain other components other than ammonia and ammonium salts, and examples of the other components include a quaternary ammonium compound, a lower alkyl amine, and the like, and optionally two or more kinds thereof. If at least one compound selected from the group consisting of a quaternary ammonium compound and a lower alkyl amine is contained in the aqueous solution, the content of at least one compound selected from the group consisting of a quaternary ammonium compound and a lower alkyl amine is usually 0.0000001 to 1 mole, preferably 0.000001 to 0.1 moles per 1 mole of ammonia. If the quaternary ammonium compound and lower alkyl amine are contained, the total content thereof may be in the range described above. The content of ammonia, ammonium salt and other components as needed in the aqueous solution may be adjusted such that the pH of the aqueous solution becomes usually 9 or more, preferably 9 to 13.

If a quaternary ammonium compound is contained in the aqueous solution, examples of the quaternary ammonium compound include hydroxides or halides of various quaternary ammoniums such as quaternary ammoniums having four groups which are independently selected from an alkyl group, an alkenyl group, an aralkyl group and an aryl group. Preferred definitions of these groups are given above. Examples of quaternary ammoniums include tetramethyl ammonium, tetraethyl ammonium, n-propyltrimethyl ammonium, tetra-n-propyl ammonium, tetra-n-butyl ammonium, triethylmethyl ammonium, tri-n-propylmethyl ammonium, tri-n-butylmethyl ammonium, benzyltrimethyl ammonium, dibenzyldimethyl ammonium, and two or more of them can be used if necessary. Among them, tetra-n-propyl ammonium compounds are preferable, and tetra-n-propyl ammonium hydroxide and tetra-n-propylammonium bromide are more preferable.

When a lower alkylamine is contained in the aqueous solution, this lower alkylamine may be a monoalkylamine, a dialkylamine, or a trialkylamine, and two or more kinds thereof can also be used if necessary. In general, a compound represented by the following formula (II) is suitably used:

$$R^5R^6R^7N \qquad (II)$$

wherein $R^5$, $R^6$, and $R^7$ each independently represents a hydrogen atom, an alkyl group having 1 to 4 carbon atoms, or an alkenyl group having 1 to 4 carbon atoms, provided that $R^5$, $R^6$, and $R^7$ are not hydrogen atoms at the same time.

Specific examples of the lower alkylamine represented by the formula (II) include alkylamines such as monomethylamine, monoethylamine, monopropylamine, monobutylamine, dimethylamine, diethylamine, dipropylamine, dibutylamine, trimethylamine, triethylamine, tripropylamine, tributylamine, etc.; allylamines such as monoallylamine, diallylamine, triallylamine, etc.; and the like, and two or more of them may be used if necessary. Among them, tripropylamine is preferred.

The contact processing with the aqueous solution may be performed in a batch system or in a continuation system. For example, the calcined product may be immersed in the aqueous solution and stirred in a stirring tank, or the aqueous solution may be allowed to flow through a tubular container in which the calcined product is filled.

The temperature of the contact processing with the aqueous solution is usually 50 to 250°C, preferably 50 to 200°C, more preferably 60 to 150°C, and the time required for the contact processing is usually 0.1 to 10 hours. In addition, the amount used of the aqueous solution is usually 100 to 5000 parts by weight based on 100 parts by weight of the calcined product.

The zeolite molded article after the contact processing with the aqueous solution is subjected to washing with water, drying, etc., if necessary. The contact processing with the aqueous solution may be carried out more than once if necessary.

The zeolite molded article after the contact processing with the aqueous solution may be subjected to a heat treatment, if necessary. The heat treatment temperature is preferably 500 to 700°C, and the time for the heat treatment is preferably 0.1 to 100 hours.

The heat treatment may be carried out under an atmosphere of an oxygen-containing gas or may be carried out under an atmosphere of an inert gas such as nitrogen and the like. The oxygen-containing gas and inert gas may contain water vapor. In addition, the heat treatment may be carried out in multiple stages under an atmosphere of an oxygen-containing gas or an inert gas. The heat treatment may be performed in a fluidized bed mode or in a fixed bed mode.

The method for producing ε-caprolactam according to the present invention comprises Beckmann rearrangement reaction of cyclohexanone oxime in a gaseous phase in the presence of a catalyst using the zeolite molded article produced by the method of the present invention. As reaction conditions for Beckmann rearrangement, the reaction temperature is usually 250 to 500°C, preferably 300 to 450°C. The reaction pressure is usually 0.005 to 0.5 MPa, preferably 0.005 to 0.2 MPa. This reaction may be performed either in a fixed bed mode or in a fluidized bed mode. The rate of feed of cyclohexanone oxime as a raw material is usually 0.1 to 20 $h^{-1}$, preferably 0.2 to 10 $h^{-1}$, as a feeding rate (kg/h) per 1 kg of catalyst, that is, as a space velocity WHSV ($h^{-1}$).

Cyclohexanone oxime may, for example, either be independently introduced into a reaction system or may be introduced together with an inert gas, such as nitrogen, argon, carbon dioxide, etc. In addition, the following methods are also effective, that is: a method in coexistence of ethers described in JP 2-250866 A; a method in coexistence of lower alcohols described in JP 2-275850 A; a method in coexistence of alcohols and/or ethers, and water described in JP 5-201965 A; a method in coexistence of ammonia described in JP 5-201966 A; and a method in coexistence of methylamine described in JP 6-107627 A.

In addition, cyclohexanone oxime may be, for example, those prepared by oximation of cyclohexanone with hydroxylamine or its salt, or those prepared by ammoximation of cyclohexanone with ammonia and hydrogen peroxide in the presence of a catalyst such as titano-silicate, etc.

Further, the Beckmann rearrangement reaction may be carried out in combination with an operation of calcining a catalyst under an atmosphere of an oxygen-containing gas such as air, etc. This calcining treatment of the catalyst enables removing by combustion of carbonaceous materials deposited on the catalyst, and can increase a conversion rate of the cyclohexanone oxime and a persistency of selectivity of the ε-caprolactam. For example, when the reaction is performed in a fixed bed mode, a method is suitably adopted wherein cyclohexanone oxime optionally together with other components is supplied into a fixed bed type reactor having a solid catalyst charged therein so that the reaction is performed, subsequently supply of cyclohexanone oxime is stopped, then calcination is carried out by supplying an oxygen-containing gas, and further these reaction and calcination are repeated. When the reaction is performed in a fluidized bed mode, a method is suitably adopted wherein while performing the reaction by supplying cyclohexanone oxime optionally with other components into a fluidized bed reactor having a solid catalyst being fluidized therein, the solid catalyst is withdrawn continuously or intermittently from the reactor and returned into the reactor again after calcined in a calcination furnace.

As post-treatment operations of the reaction mixture obtained by the Beckmann rearrangement reaction, known methods can be appropriately employed, and for example, after cooling and condensation of the gas produced by the reaction, ε-caprolactam can be separated by performing operations of extraction, distillation, crystallization, etc.

EXAMPLES

Hereinafter, Examples of the present invention will be described, but the present invention is not limited thereto. In addition, the space velocity WHSV ($h^{-1}$) of cyclohexanone oxime was calculated by dividing a feeding rate (kg/h) of cyclohexanone oxime by a catalyst weight (kg). Analysis of cyclohexanone oxime and ε-caprolactam was conducted using gas chromatography, and a conversion rate of cyclohexanone oxime and a selectivity of ε-caprolactam were calculated by the following equations, respectively, where the number of moles of supplied cyclohexanone oxime was defined as X, the number of moles of unreacted cyclohexanone oxime as Y, and the number of moles of produced ε-caprolactam as Z.

Measurement of the pulverization rate of a zeolite molded article obtained in each Example below was carried out according to the following method.

$$\text{Conversion rate of cyclohexanone oxime (\%)} = [(X - Y)/X] \times 100$$

$$\text{Selectivity of } \varepsilon\text{-caprolactam (\%)} = [Z/(X - Y)] \times 100$$

Measurement of pulverization rate

A zeolite molded article 50 g was allowed to flow in a stainless steel column (127 mmφ, 559 mm in length) while supplying an air (7 liters/h) from the bottom for 20 hours, during which time a pulverization rate of the molded article was measured. The pulverization rate was measured by the following equation wherein the weight (g) of zeolite molded article initially filled was defined as x, the weight (g) of pulverized fine particles which had come out from the upper part of the column until 5 hours after the start of air supply was defined as y, and the weight (g) of pulverized fine particles which had come out from the upper part of the column from 5 to 20 hours after the start of air supply was defined as z. In addition, the pulverization rate corresponds to an abrasion resistance of the molded article, and the smaller this value is, the higher abrasion resistance is, indicating that such article is excellent in mechanical strength.

$$\text{Pulverization rate (\%)} = [z/(x - y)] \times 100$$

Example 1

Production of zeolite molded article

Hydrothermal synthesis and washing

Into a 30L-stainless steel autoclave were placed tetraethyl orthosilicate $[Si(OC_2H_5)_4]$ 5208 g, 40% by weight aqueous tetra-n-propylammonium hydroxide solution 3246 g, potassium hydroxide (purity: 85%) 50.7 g and water 14288 g, and the mixture was stirred vigorously at room temperature for 120 minutes. The molar ratios of water, tetra-n-propylammonium ions, hydroxide ions, and potassium ions to silicon in the resulting mixture were 36, 0.248, 0.278, and 0.048, respectively. This mixture was stirred at 105°C and 300 rpm for 48 hours to perform a hydrothermal synthesis reaction. The obtained reaction mixture was subjected to filtration in a cross-flow manner to separate a concentrate containing crystals and a filtrate. The obtained concentrate containing crystals was washed with water and filtered in a cross-flow manner, and washing was repeated until the pH of the washing filtrate became 9.03 (25°C) . After washing, a slurry containing crystals [content of the crystals (excluding a portion of the structure-directing agent incorporated into the crystals): 54% by mass] was collected in 2508 g.

Molding and calcination

The slurry containing crystals obtained above was spray-dried with an atomizer type spray dryer to form fine particles. The average inlet temperature and the average outlet temperature of the spray dryer were 210°C and 90°C, respectively. Using a rotary kiln, the white fine particles obtained were calcined at 530°C in a nitrogen flow for one hour, and further calcined at 530°C in a mixed gas flow of nitrogen and air [nitrogen : air = 4 : 1 (volume ratio)], thereby to obtain a white calcined product. The average particle size of the calcined product obtained was 59 $\mu$m.

Contact treatment with aqueous solution

Then, the calcined product 10 g obtained above was placed into a petri dish and hanged in a 1-liter autoclave containing 100 g of water. After closing the lid, the autoclave was placed into a thermostatic chamber at 80°C and allowed to stand for 3 hours. After removing the autoclave from the chamber, it was allowed to cool to 20°C. This solid 10 g was placed into an autoclave, and a mixed solution 278 g of 7.5% by mass aqueous ammonium nitrate solution 110 g and 25% by mass aqueous ammonia solution 168 g was added thereto. The mixture was stirred at 90°C for one hour, and filtered to separate a solid. This solid was further treated twice with a mixture of aqueous ammonium nitrate solution and aqueous ammonia solution in the same manner as described above, and then washed and dried to obtain a zeolite molded article (A). The pulverization rate of the obtained zeolite molded article (A) was 3.1%.

Production of $\varepsilon$-caprolactam

The zeolite molded article (A) 0.375 g obtained above was charged into a quartz glass reactor tube having an inner diameter of 1 cm to form a catalyst layer, and this was preheated at 350°C for one hour in a nitrogen flow of 4.2 L/h. Subsequently, after lowering the temperature of the catalyst layer to 325°C in a nitrogen flow of 4.2 L/h, a mixture of vaporized cyclohexanone oxime/methanol = 1/1.8 (weight ratio) was supplied to the reactor tube at a feeding rate of 8.4 g/h (WHSV=8 h$^{-1}$ of cyclohexanone oxime) to be reacted. A reaction gas after 5.5 hours to 5.75 hours from the start of the reaction was sampled, and analyzed using gas chromatography. A conversion rate of cyclohexanone oxime was 99.8%, and a selectivity of $\varepsilon$-caprolactam was 96.8%.

Example 2

Production of zeolite molded article

Hydrothermal synthesis and washing

Into a 30L-stainless steel autoclave were placed tetraethyl orthosilicate [$Si(OC_2H_5)_4$] 5208 g, 40% by weight aqueous tetra-n-propylammonium hydroxide solution 2618 g, potassium hydroxide (purity: 85%) 56.2 g and water 14661 g, and the mixture was stirred vigorously at room temperature for 120 minutes. The molar ratios of water, tetra-n-propylammonium ions, hydroxide ions, and potassium ions to silicon in the resulting mixture were 36, 0.200, 0.234, and 0.048, respectively. This mixture was stirred at 105°C and 300 rpm for 48 hours to perform a hydrothermal synthesis reaction. The obtained reaction mixture was subjected to filtration in a cross-flow manner to separate a concentrate containing crystals and a filtrate. The obtained concentrate containing crystals was washed with water and filtered in a cross-flow manner, and washing was repeated until the pH of the washing filtrate became 9.01 (25°C). After washing, a slurry containing crystals [content of the crystals (excluding a portion of the structure-directing agent incorporated into the crystals): 57% by mass] was collected in 2224 g.

Molding and calcination

The slurry containing crystals obtained above was treated in the same manner as [Molding and calcination] of Example 1, thereby to obtain a white calcined product. The average particle size of the calcined product obtained was 64 $\mu$m.

Contact treatment with aqueous solution

The calcined product obtained above was treated in the same manner as [Contact treatment with aqueous solution] of Example 1, thereby to obtain a zeolite molded article (B). The pulverization rate of the obtained zeolite molded article (B) was 2.7%.

Production of ε-caprolactam

The same procedure as (Production of ε-caprolactam) of Example 1 was carried out, except that the zeolite molded article (B) was used in place of the zeolite molded article (A). The conversion of cyclohexanone oxime was 99.3% and the selectivity of ε-caprolactam was 96.8%.

Comparative Example 1

Production of zeolite molded article

Hydrothermal synthesis and Washing

Into a 30L-stainless steel autoclave were placed tetraethyl orthosilicate $[Si(OC_2H_5)_4]$ 5208 g, 40% by weight aqueous tetra-n-propylammonium hydroxide solution 3246 g, potassium hydroxide (purity: 85%) 50.7 g and water 14288 g, and the mixture was stirred vigorously at room temperature for 120 minutes. The molar ratios of water, tetra-n-propylammonium ions, hydroxide ions, and potassium ions to silicon in the resulting mixture were 36, 0.248, 0.278, and 0.048, respectively. This mixture was stirred at 105°C and 300 rpm for 48 hours to perform a hydrothermal synthesis reaction. The obtained reaction mixture was subjected to a cross-flow filtration to separate a concentrate containing crystals and a filtrate, The obtained concentrate containing crystals was washed with water and filtered in a cross-flow manner, and washing was repeated until the pH of the washing filtrate became 7.98 (25°C). After washing, a slurry containing crystals [content of the crystals (excluding a portion of the structure-directing agent incorporated into the crystals): 52% by mass] was collected in 2213 g.

Molding and calcination

The slurry containing crystals obtained above was treated in the same manner as [Molding and calcination] of Example 1, thereby to obtain a white calcined product. The average particle size of the calcined product obtained was 54 μm.

Contact treatment with aqueous solution

The calcined product obtained above was treated in the same manner as [Contact treatment with aqueous solution] of Example 1, thereby to obtain a zeolite molded article (C). The pulverization rate of the obtained zeolite molded article (C) was 7.4%.

Production of ε-caprolactam

The same procedure as (Production of ε-caprolactam) of Example 1 was carried out, except that the zeolite molded article (C) was used in place of the zeolite molded article (A). The conversion of cyclohexanone oxime was 99.9% and the selectivity of ε-caprolactam was 96.7%.

Comparative Example 2

Production of zeolite molded article

Hydrothermal synthesis and washing

Into a 30L-stainless steel autoclave were added tetraethyl orthosilicate $[Si(OC_2H_5)_4]$ 5208 g, 40% by weight aqueous tetra-n-propylammonium hydroxide solution 2618 g, potassium hydroxide (purity: 85%) 56.2 g and water 14661 g, and the mixture was stirred vigorously at room temperature for 120 minutes. The molar ratios of water, tetra-n-propylammonium ions, hydroxide ions, and potassium ions to silicon in the resulting mixture were 36, 0.200, 0.234, and 0.048, respectively. This mixture was stirred at 105°C and 300 rpm for 48 hours to perform a hydrothermal synthesis reaction. The obtained reaction mixture was subjected to

filtration in a cross - flow manner to separate a concentrate containing crystals and a filtrate. The obtained concentrate containing crystals was washed with water and filtered in a cross-flow manner, and washing was repeated until the pH of the washing filtrate became 8.10 (25°C). After washing, a slurry containing crystals [content of the crystals (excluding a portion of the structure-directing agent incorporated into the crystals): 55% by mass] was collected in 2245 g.

Molding and calcination

The slurry containing crystals obtained above was treated in the same manner as [Molding and calcination] of Example 1, thereby to obtain a white calcined product. The average particle size of the calcined product obtained was 64 $\mu$m.

Contact treatment with aqueous solution

The calcined product obtained above was treated in the same manner as [Contact treatment with aqueous solution] of Example 1, thereby to obtain a zeolite molded article (D) . The pulverization rate of the obtained zeolite molded article (D) was 8.4%.

Production of $\varepsilon$-caprolactam

The same procedure as (Production of $\varepsilon$-caprolactam) of Example 1 was carried out, except that the zeolite molded article (D) was used in place of the zeolite molded article (A). The conversion of cyclohexanone oxime was 99.6% and the selectivity of $\varepsilon$-caprolactam was 96.7%.

**Claims**

1. A method for producing a zeolite molded article, comprising the following steps (1) to (7):

    (1) subjecting a mixture containing a silicon compound, water, and a quaternary ammonium hydroxide to a hydrothermal synthesis reaction;
    (2) filtering the reaction mixture containing crystals obtained in the step (1) to separate it into a concentrate containing crystals and a filtrate;
    (3) washing the concentrate containing crystals obtained in the step (2) with water until the pH at 25°C of the washed solution obtained by washing becomes 8.5 to 9.5;
    (4) molding the crystals obtained after washing in the step (3);
    (5) calcining the molded article obtained in the step (4);
    (6) contacting the calcined product obtained in the step (5) with water vapor; and
    (7) contact treating the calcined product obtained after contact with water vapor in the step (6) with an aqueous solution including at least one compound selected from the group consisting of ammonia and an ammonium salt.

2. The method according to claim 1, wherein the step (3) is the following step (3'):
    (3') washing and filtering the concentrate containing crystals obtained in the step (2) in a cross flow manner with water until the pH at 25°C of the washing filtrate becomes 8.5 to 9.5,
    and the washed crystals obtained in the step (3') are subjected to the step (4).

3. The method according to claim 1 or 2, wherein the molding in the step (4) is carried out by spray drying a slurry that contains the washed crystals.

4. The method according to any one of claims 1 to 3, wherein at least one of at least a portion of the filtrate obtained in the step (2) and at least a portion of the washed solution obtained by washing in the step (3) is further mixed in the step (1).

5. The method according to any one of claims 1 to 4, wherein the zeolite is a pentasil type zeolite.

6. A method for producing $\varepsilon$-caprolactam, comprising producing a zeolite molded article by the method according to any one of claims 1 to 5 and subjecting cyclohexanone oxime to Beckmann rearrangement reaction in a gaseous phase in the presence of the zeolite molded article.

**Patentansprüche**

1. Ein Verfahren zur Herstellung eines Zeolithformgegenstands, umfassend die folgenden Schritte (1) bis (7):

(1) Unterziehen eines Gemischs, welches eine Siliciumverbindung, Wasser und ein quaternäres Ammoniumhydroxid enthält, einer hydrothermalen Synthesereaktion;

(2) Filtrieren des in Schritt (1) erhaltenen Reaktionsgemisches, das Kristalle enthält, um es in ein Konzentrat, das Kristalle enthält, und ein Filtrat zu trennen;

(3) Waschen des in Schritt (2) erhaltenen Konzentrats, das Kristalle enthält, mit Wasser, bis der pH-Wert bei 25°C der durch Waschen erhaltenen gewaschenen Waschlösung 8,5 bis 9,5 beträgt;

(4) Formen der in Schritt (3) nach dem Waschen erhaltenen Kristalle;

(5) Kalzinieren des in Schritt (4) erhaltenen Formgegenstands;

(6) In Kontakt bringen des in Schritt (5) erhaltenen kalzinierten Erzeugnisses mit Wasserdampf; und

(7) Kontaktbehandeln des in Schritt (6) nach Kontakt mit dem Wasserdampf erhaltenen kalzinierten Erzeugnisses mit einer wässrigen Lösung, die mindestens eine Verbindung ausgewählt aus der Gruppe bestehend aus Ammoniak und einem Ammoniumsalz enthält.

2. Das Verfahren gemäß Anspruch 1, wobei der Schritt (3) der nachfolgende Schritt (3') ist:
(3') Waschen und Filtrieren des in Schritt (2) erhaltenen Konzentrats, das Kristalle enthält, kreuzstrommäßig mit Wasser, bis der pH-Wert bei 25°C des Waschfiltrats 8,5 bis 9,5 beträgt,
und wobei die in Schritt (3') erhaltenen gewaschenen Kristalle dem Schritt (4) unterzogen werden.

3. Das Verfahren gemäß Anspruch 1 oder 2, wobei das Formen in Schritt (4) mittels Sprühtrocknen einer Aufschlämmung, die die gewaschenen Kristalle enthält, durchgeführt wird.

4. Das Verfahren gemäß einem der Ansprüche 1 bis 3, wobei mindestens eines von mindestens einem Teil des in Schritt (2) erhaltenen Filtrats und mindestens einem Teil der durch Waschen in Schritt (3) erhaltenen Waschlösung in Schritt (1) weiter gemischt wird.

5. Das Verfahren gemäß einem der Ansprüche 1 bis 4, wobei der Zeolith ein Zeolith vom Pentasiltyp ist.

6. Ein Verfahren zur Herstellung von ε-Caprolactam, umfassend das Herstellen eines Zeolithformgegenstands durch das Verfahren gemäß einem der Ansprüche 1 bis 5 und Unterziehen von Cyclohexanonoxim einer Beckmann-Umlagerungsreaktion in einer gasförmigen Phase in Gegenwart des Zeolithformgegenstands.

**Revendications**

1. Méthode pour produire un article moulé en zéolite, comprenant les étapes (1) à (7) suivantes :

(1 soumission d'un mélange contenant un composé du silicium, de l'eau, et un hydroxyde d'ammonium quaternaire à une réaction de synthèse hydrothermale ;

(2) filtration du mélange réactionnel contenant des cristaux obtenu dans l'étape (1) pour le séparer en un concentré contenant des cristaux et un filtrat ;

(3) lavage du concentré contenant des cristaux obtenu dans l'étape (2) avec de l'eau jusqu'à ce que le pH à 25°C de la solution lavée obtenue par lavage devienne de 8,5 à 9,5 ;

(4) moulage des cristaux obtenus après lavage dans l'étape (3) ;

(5) calcination de l'article moulé obtenu dans l'étape (4) ;

(6) mise en contact du produit calciné obtenu dans l'étape (5) avec de la vapeur d'eau ; et

(7) traitement du produit calciné obtenu après contact avec de la vapeur d'eau dans l'étape (6), par contact avec une solution aqueuse contenant au moins un composé choisi dans le groupe constitué par l'ammoniac et un sel d'ammonium.

2. Méthode selon la revendication 1, dans laquelle l'étape (3) est l'étape (3') suivante :
(3') lavage et filtration du concentré contenant des cristaux obtenu dans l'étape (2) d'une manière à courants croisés avec de l'eau jusqu'à ce que le pH à 25°C de la solution lavée obtenue par lavage devienne de 8,5 à 9,5,
et les cristaux lavés obtenus dans l'étape (3') sont soumis à l'étape (4).

**3.** Méthode selon la revendication 1 ou 2, dans laquelle le moulage dans l'étape (4) est effectué par séchage par pulvérisation d'une bouillie qui contient les cristaux lavés.

**4.** Méthode selon l'une quelconque des revendications 1 à 3, dans laquelle au moins l'une parmi au moins une partie du filtrat obtenu dans l'étape (2) et au moins une partie de la solution lavée obtenue par lavage dans l'étape (3) est en outre mélangée dans l'étape (1).

**5.** Méthode selon l'une quelconque des revendications 1 à 4, dans laquelle la zéolite est une zéolite de type pentasil.

**6.** Méthode pour produire de l'ε-caprotactame, comprenant la production d'un article moulé en zéolite par la méthode de l'une quelconque des revendications 1 à 5 et la soumission de cyclohexanone oxime à une réaction de réarrangement de Beckmann en phase gazeuse en présence de l'article moulé en zéolite.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2250866 A
- JP 2275850 A
- JP 6072706 A
- JP 5201965 A
- JP 5201966 A
- JP 6107627 A

**Non-patent literature cited in the description**

- Science and Engineering of Zeolite (in Japanese). Kodansha Scientific Ltd, 2000, 33-34